# EUROPEAN PATENT APPLICATION

(11) **EP 3 705 136 A1**
(43) Date of publication of application: **09.09.2020**
(21) Application number: 18872316.7
(22) Date of filing: 29.10.2018
(51) Int. Cl.: A61K 45/00, A61K 9/08, A61K 31/444, A61K 47/10, A61K 47/14, A61K 47/18, A61P 17/00, A61P 31/10

(54) **EXTERNAL PREPARATION FOR TREATING TRICHOPHYTOSIS UNGUIUM**

(30) Priority: 30.10.2017 JP 2017209136
(71) Applicant: Kaken Pharmaceutical Co., Ltd., Tokyo 113-8650 (JP)
(72) Inventor: NATORI, Nobuyuki, Fujieda-shi Shizuoka 426-8646 (JP); TAKABE, Hiroyuki, Fujieda-shi Shizuoka 426-8646 (JP); ISHIMARU, Takashi, Fujieda-shi Shizuoka 426-8646 (JP); ISEKI, Hiroshi, Fujieda-shi Shizuoka 426-8646 (JP); KARASAWA, Keiichi, Fujieda-shi Shizuoka 426-8646 (JP)
(74) Representative: J A Kemp LLP
(86) International application number: PCT/JP2018/040047
(87) International publication number: WO 2019/088005

(57) **Abstract**

The present invention provides a non-coating topical therapeutic agent for tinea unguium containing medium-chain triglyceride and the like as non-volatile components, which agent has high nail permeability and is superior in the aspects of efficacy and preparation properties, and can be applied clinically. Particularly, the present invention provides a topical therapeutic agent for tinea unguium containing an antifungal active substance such as efinaconazole or the like, a volatile component, an additive such as medium-chain triglyceride or the like, and a permeation enhancer such as ethyl lactate and the like. The preparation shows remarkably improved nail permeability has superior properties as a pharmaceutical product from the aspects of efficacy and preparation properties.

## Description

### [Technical Field]

The present invention relates to a topical pharmaceutical preparation effective for tinea unguium.

### [Background Art]

As topical preparations used for treating tinea unguium, efinaconazole, luliconazole, amorolfine and the like have already been placed on the market to date. However, a therapeutic agent having a higher treatment effect on tinea unguium is demanded.

Topical preparations used for treating tinea unguium are superior to oral preparations in that they cause fewer systemic side effects and have less drug-drug interactions. To exhibit a sufficient pharmacological effect, the drug needs to sufficiently penetrate into the thick stratum corneum of the nail plate, and exert an antifungal activity against Trichophyton fungus in nails and on the nail bed (see, for example, non-patent document 1).

To efficiently deliver the active pharmaceutical ingredient to a lesion on the nail bed, the design of a pharmaceutical formulation is extremely important. The nail permeability of the antifungal active substance greatly varies depending on the properties of the antifungal active substance itself, as well as the properties of each formulation component and the mixing ratio thereof. Formulation components also affect efficacy, texture after application, odor, and the like. Thus, the pharmaceutical formulation should be designed in consideration of their balance.

Preparations of topical therapeutic agents for tinea unguium are mainly divided into coating preparations and non-coating preparations.

In both cases of coating preparations and non-coating preparations, nail permeability of the antifungal active substance is extremely important for the expression of clinical efficacy.

In coating preparations, the coating formed on the nail surface often suppresses diffusion of the antifungal active substance, and prevents release of the antifungal active substance from the coating and penetration thereof into the stratum corneum. In such cases, even if the coating preparation is repeatedly applied to the nail, the coating applied previously prevents penetration of the antifungal active substance in the newly-applied coating, and thus the effect is weak even when used continuously.

In the case of non-coating topical therapeutic agents for tinea unguium, a "volatile component" that evaporates quickly after application to the affected part and a "non-volatile component" that remains in the affected part and dissolves the antifungal active substance need to be contained as additives. When selection of the "non-volatile component" is improper, the antifungal active substance is precipitated in the applied part and the nail permeability is lost.

Medium-chain triglyceride is widely used as an additive for pharmaceutical products and foods. Medium-chain triglyceride has a precedent of use as a base for topical pharmaceutical preparations in clinical use, and is widely used as a base for, for example, liquid, ointment, cream, liquid suspension and the like (see, for example, non-patent document 2, non-patent document 3).

Therefore, medium-chain triglyceride is one of the most appropriate components as a "non-volatile component" in designing the pharmaceutical formulation of a non-coating topical therapeutic agent for tinea unguium.

As other examples in which medium-chain triglyceride is used as a base of topical preparations, patent document 1 and patent document 2 can be mentioned.

However, these documents do not disclose a topical preparation useful for treating tinea unguium, not to mention these documents neither disclose nor suggest a method for improving nail permeability and efficacy of an topical preparation containing a physiologically active substance such as an antifungal active substance or the like and a non-volatile component such as medium-chain triglyceride or the like as an additive.

### [Document List]

### [Patent documents]

Patent document 1: JP-A-2008-019197
Patent document 2: WO 2009/028495

### [Non-patent documents]

Non-patent document 1: Clinical manual of Fungal nail disease (2005), NANKODO; 109-112
Non-patent document 2: Astat (registered trade mark) Cream 1% Pharmaceutical Interview Forms, Maruho Co., Ltd. June, 2016 (revised 7th edition)
Non-patent document 3: ZEFNART (registered trade mark) CREAM 2% Pharmaceutical Interview Forms, Torii Pharmaceutical Co., Ltd. January, 2010 (revised 7th edition)

### [SUMMARY OF THE INVENTION]

### [Problems to be Solved by the Invention]

In view of the above-mentioned background, the present invention aims to provide a non-coating topical therapeutic agent for nail diseases (particularly, topical therapeutic agent for tinea unguium) containing medium-chain triglyceride and the like as non-volatile components. Such topical therapeutic agent for nail diseases (particularly, topical therapeutic agent for tinea unguium) is superior in the aspects of nail permeability, efficacy, and preparation properties, and can be applied clinically.

### [Means of Solving the Problems]

In view of the above-mentioned problem, the inventors have conducted intensive studies and found that a preparation having superior properties as an topical therapeutic agent for nail diseases (particularly, topical therapeutic agent for tinea unguium) in terms of nail permeability, efficacy, and preparation properties can be obtained by further adding a permeation enhancer such as ethyl lactate or the like to a formulation containing a physiologically active substance such as an antifungal active substance or the like and a non-volatile component such as medium-chain triglyceride or the like, which resulted in the completion of the present invention.

That is, the present invention provides the following.
[1] A topical preparation that is a therapeutic agent for tinea unguium, comprising:
   a) an antifungal active substance,
   b) a volatile component,
   c) a medium-chain triglyceride, and
   d) ethyl lactate.
[2] The topical preparation of [1], wherein an amount of the ethyl lactate is 1 w/w% to 30 w/w%.
[3] The topical preparation of [1] or [2], wherein an amount of the ethyl lactate is 5 w/w% to 22 w/w%.
[4] The topical preparation of any of [1] to [3], wherein an amount of the ethyl lactate is 10 w/w% to 22 w/w%.
[5] The topical preparation of any of [1] to [4], wherein an amount of the medium-chain triglyceride is 1 w/w% to 30 w/w%.
[6] The topical preparation of any of [1] to [5], wherein an amount of the medium-chain triglyceride is 5 w/w% to 22 w/w%.
[7] The topical preparation of any of [1] to [6], wherein an amount of the medium-chain triglyceride is 10 w/w% to 22 w/w%.
[8] The topical preparation of any of [1] to [7], wherein an amount of the antifungal active substance is 1 w/w% to 30 w/w%.
[9] The topical preparation of any of [1] to [8], wherein an amount of the antifungal active substance is 1 w/w% to 15 w/w%.
[10] The topical preparation of any of [1] to [9], wherein the volatile component is ethanol.
[11] The topical preparation of [10], wherein an amount of the ethanol is 35 w/w% to 85 w/w%.
[12] The topical preparation of any of [1] to [11], wherein an acid value of the ethyl lactate is 0 to 0.5.
[13] The topical preparation of any of [1] to [12], further comprising e) disodium edetate hydrate.
[14] The topical preparation of [13], wherein an amount of the disodium edetate hydrate is 0.0001 w/w% to 0.001 w/w%.
[15] The topical preparation of any of [1] to [14], wherein the antifungal active substance is a compound represented by the formula (I): 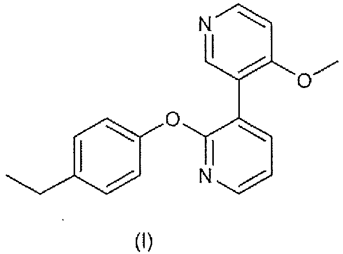 or a salt thereof.
[16] The topical preparation of any of [1] to [14], wherein the antifungal active substance is a compound represented by the formula (II): 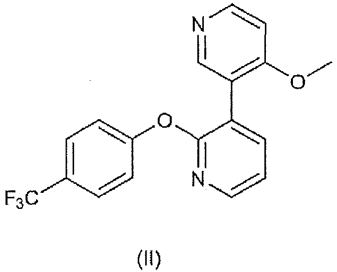 or a salt thereof.
[17] The topical preparation of any of [1] to [14], wherein the antifungal active substance is efinaconazole or a salt thereof.
[18] The topical preparation of any of [1] to [14], wherein the antifungal active substance is luliconazole or a salt thereof.

### [Effect of the Invention]

According to the present invention, a topical therapeutic agent for nail diseases (particularly, topical therapeutic agent for tinea unguium) which is superior in the aspects of nail permeability, efficacy, and preparation properties, and can be applied clinically can be provided.

### [Description of Embodiments]

The respective terms in the present specification are explained below.

The "physiologically active substance" is a substance used for treating some pathology, and exemplified by antifungal active substances and the like.

The "antifungal active substance" is a low-molecular-weight compound that shows a fungistatic or fungicidal antifungal activity against pathogenic fungus of tinea unguium such as Trichophyton rubrum, Trichophyton mentagrophytes and the like. The "antifungal active substance" in the present specification is not particularly limited as long as it is a compound showing such antifungal activities. Preferable examples include azole antifungal agents such as efinaconazole, luliconazole, clotrimazole, croconazole, econazole, ketoconazole, neticonazole, itraconazole and the like, allylamine antifungal agents such as terbinafine and the like, biaryl compounds shown in WO 2017/047602, 2-(1H-pyrazol-1-yl)phenol derivatives shown in WO 2012/102404, amorolfine and the like.

It is further preferably the biaryl compound shown in WO 2017/047602 or a salt thereof, particularly preferably the biaryl compound shown in WO 2017/047602 and represented by the following formula (I) or (II) 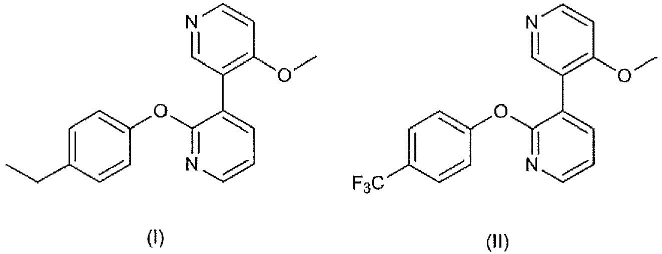 or a salt thereof, and particularly preferably a biaryl compound represented by the above-mentioned formula (I) (hereinafter to be also referred to as "compound A") or a salt thereof.

A salt of the biaryl compound represented by the formula (I) or (II) is preferably a pharmacologically acceptable salt. The "pharmacologically acceptable salt" is not particularly limited as long as it is a pharmacologically acceptable salt. Examples thereof include inorganic acid salts such as hydrochloride, hydrobromide, nitrate, sulfate, phosphate and the like, organic carboxylic acid salts such as acetate, oxalate, fumarate, maleate, malonate, citrate, succinate, lactate, tartrate, malate and the like, aromatic carboxylic acid salts such as salicylate, benzoate and the like, organic sulfonic acid salts such as methanesulfonate, benzenesulfonate and the like, and other salts.

In another embodiment of the present invention, a preferable antifungal active substance is efinaconazole, luliconazole or terbinafine, and a more preferable antifungal active substance is efinaconazole or luliconazole.

The content of the "antifungal active substance" contained in the preparation of the present invention is not particularly limited. It is preferably 1 w/w% to 30 w/w%, more preferably 1 w/w% to 15 w/w%, still more preferably 5 w/w% to 15 w/w%, and a particularly preferable content is 8 w/w% to 12 w/w%.

In another embodiment of the present invention, the content of the "antifungal active substance" contained in the preparation of the present invention is not particularly limited. It is preferably 1 w/w% to 30 w/w%, more preferably 1 w/w% to 15 w/w%, still more preferably 1 w/w% to 12 w/w%, and a particularly preferable content is 2 w/w% to 12 w/w%.

The "coating preparation" refers to a topically application preparation that forms an aqueous or water-insoluble coating film with good stability after being applied to the nail. The characteristic of the coating preparation is that, due to good adhesiveness, a physiologically active substance such as an antifungal active substance or the like can be stably attached to the nail for a long time.

Specific examples of the coating preparation include nail lacquer preparations, nail enamel preparations, manicure preparations and the like.

The coating preparation contains a film-forming substance. As the film-forming substance, water-soluble polymers such as copolyvidone, povidone, poly(vinyl alcohol) and the like, water-insoluble polymers such as copolymer of acrylate ester and methacrylate ester, pyroxylin, methyl methacrylate-butyl methacrylate-dimethylaminoethyl methacrylate-copolymer, polyvinylacetal diethylamino acetate, nitrocellulose, hydroxypropylchitosan and the like are known.

The "non-coating preparation" refers to a topically application preparation substantially free of the above-mentioned film-forming substance in the preparation.

Being "substantially free of" in this case means that a coating is not substantially formed on the surface of the nail after applying even when a film-forming substance is contained in the preparation.

In the case of a non-coating topical preparation, to prevent precipitation of a physiologically active substance such as an antifungal active substance or the like after applying the drug to a nail, the preparation needs to substantially contain a non-volatile component as an additive.

In this case, "substantially contain" means containing a non-volatile component in an amount free from precipitation of a physiologically active substance such as an antifungal active substance or the like at the application site.

The "volatile component" is not particularly limited as long as it evaporates rapidly at ordinary temperature after applying a drug to a human nail. One that has a precedent of clinical use as a topical preparation, is industrially easily available, and odorless and nonstimulating is preferable. Preferably, it is a straight chain or branched chain lower alkyl alcohol having 1 - 4 carbon atoms such as ethanol, isopropanol or the like, more preferably ethanol.

When ethanol is used as a "volatile component", water-containing ethanol (e.g., 95 vol% ethanol) can also be used as long as ethyl lactate is not hydrolyzed; however, anhydrous ethanol is more preferable.

The content of the "volatile component" to be contained in the preparation of the present invention is not particularly limited as long as a physiologically active substance such as an antifungal active substance and the like and other additive are dissolved. It is preferably 35 w/w% to 85 w/w%, more preferably 35 w/w% to 65 w/w%, and particularly preferably 45 w/w% to 65 w/w%.

In another embodiment of the present invention, the content of the "volatile component" to be contained in the preparation of the present invention is not particularly limited as long as a physiologically active substance such as an antifungal active substance and the like and other additive are dissolved. It is preferably 35 w/w% to 85 w/w%, more preferably 35 w/w% to 75 w/w%, and particularly preferably 45 w/w% to 75 w/w%.

The "additive" refers to a base or an additive substance suitable as a topically application preparation. In the present invention, a preferable additive is a non-volatile component, more preferably a medium-chain triglyceride.

The "medium-chain triglyceride" is a non-volatile component in which 3 molecules of fatty acid are esterified with one molecule of glycerol and the fatty acids are saturated fatty acids having 6 - 14 carbon atoms. A preferable carbon number of the fatty acid is 8 - 12 and, for example, caprylic acid, capric acid, lauric acid or the like is selected.

A preferable medium-chain triglyceride includes caprylic acid triglyceride, capric acid triglyceride, a mixture of caprylic acid triglyceride and capric acid triglyceride, a mixture of caprylic acid triglyceride, capric acid triglyceride and lauric acid triglyceride, tri(caprylic acid/capric acid) glyceride and the like. For example, MIGLYOL (registered trade mark) 810 and 812 and the like can be used.

A particularly preferable "medium-chain triglyceride" of the present invention is tri(caprylic acid/capric acid) glyceride.

In the case of non-coating preparations, additives such as medium-chain triglyceride and the like are added to prevent precipitation of a physiologically active substance such as an antifungal active substance or the like after applying a non-coating preparation to a nail. Therefore, the content is not particularly limited as long as an antifungal active substance is not precipitated after applying. The content of an additive such as medium-chain triglyceride and the like to be contained in the preparation of the present invention is preferably 1 w/w% to 30 w/w%, more preferably 5 w/w% to 22 w/w%, still more preferably 10 w/w% to 22 w/w%. A particularly preferable content is 18 w/w% to 22 w/w%, and an optimal content is about 20 w/w%.

The "permeation enhancer" is used for permeation of a physiologically active substance such as an antifungal active substance or the like into a nail. Addition of a permeation enhancer to a preparation can achieve rapid transfer of a large amount of a physiologically active substance into a nail. Examples of the permeation enhancer include, but are not limited to, lower (C1-C4) alkyl ester of hydroxyl acid such as ethyl lactate and the like, aliphatic (C8-C20) alcohol ester of hydroxyl acid such as lauryl lactate and the like, organic acids such as lactic acid and the like, sulfonic acids such as sodium dodecylsulfonate and the like, sulfoxides such as dimethyl sulfoxide, decylmethyl sulfoxide and the like, acid amides such as pyrrolidone and the like, polyalcohols such as propylene glycol and the like, N-acetyl-L-cysteine, salicylic acid, urea, stratum corneum dissolving agent and the like.

In the present invention, a preferable permeation enhancer is lower (C1-C4) alkyl ester of hydroxyl acid, more preferably lower (C1-C4) alkyl ester of aliphatic hydroxyl acid, further preferably C1-C4 alkyl lactate, and a particularly preferable permeation enhancer is ethyl lactate.

The aforementioned "hydroxyl acid" refers to an organic acid having a hydroxyl group and a carboxyl group, and specifically includes aliphatic hydroxyl acids such as lactic acid, malic acid, tartaric acid, citric acid and the like, and aromatic hydroxyl acids such as salicylic acid and the like.

The "C1-C4 alkyl lactate" is a lower (C1-C4) alkyl ester of lactic acid, and specifically includes methyl lactate, ethyl lactate, n-propyl lactate, n-butyl lactate and the like.

The "ethyl lactate" is an ethyl ester of lactic acid. Examples of the lactic acid include L-lactic acid, D-lactic acid and DL-lactic acid. A preferable ethyl lactate is an ethyl ester of DL-lactic acid.

Generally, "ethyl lactate" contains peroxide, lactic acid and the like as impurities. In consideration of the stability and the like of the antifungal active substance contained in the topical preparation of the present invention, impurities are preferably as small as possible. More preferable ethyl lactate is ethyl lactate having an acid value of 0 to 0.5.

The acid value is the amount (mg) of potassium hydroxide (KOH) necessary for neutralizing 1 g of a sample. The acid value can be measured according to the Japanese Pharmacopoeia 17th Edition, General Tests, Fats and Fatty Oils Test, acid value. It can be simply measured using Simple Pack for fats and oils deterioration measurement Acid Value 1 (trade name) manufactured by SIBATA SCIENTIFIC TECHNOLOGY LTD.

The content of the permeation enhancer such as ethyl lactate and the like to be contained in the preparation of the present invention is preferably 1 w/w% to 30 w/w%, more preferably 5 w/w% to 22 w/w%, still more preferably 10 w/w% to 22 w/w%, particularly preferably 18 w/w% to 22 w/w%, and an optimal content is about 20 w/w%.

In one embodiment of the present invention, the preparation of the present invention is a non-coating preparation.

In one embodiment of the present invention, the preparation of the present invention containing an additive and a permeation enhancer in combination is a non-coating preparation superior in preparation property and nail permeability, and exhibiting a tinea unguium treatment effect.

A preferable combination of the additive and the permeation enhancer in the present invention is shown by a non-coating preparation containing a non-volatile component and a permeation enhancer in combination.

As a combination of an additive and a permeation enhancer contained in the topical preparation of the present invention, a combination of the above-mentioned preferable additive and the above-mentioned preferable permeation enhancer is preferable. For example, the following combinations are preferable.
(1) Combination of 1 w/w% to 30 w/w% of additive and 1 w/w% to 30 w/w% of permeation enhancer.
(2) Combination of 5 w/w% to 22 w/w% of additive and 5 w/w% to 22 w/w% of permeation enhancer.
(3) Combination of 10 w/w% to 22 w/w% of additive and 5 w/w% to 22 w/w% of permeation enhancer.
(4) Combination of 18 w/w% to 22 w/w% of additive and 5 w/w% to 22 w/w% of permeation enhancer.
(5) Combination of 20 w/w% of additive and 5 w/w% to 22 w/w% of permeation enhancer.
(6) Combination of 5 w/w% to 22 w/w% of additive and 10 w/w% to 22 w/w% of permeation enhancer.
(7) Combination of 5 w/w% to 22 w/w% of additive and 18 w/w% to 22 w/w% of permeation enhancer.
(8) Combination of 5 w/w% to 22 w/w% of additive and 20 w/w% of permeation enhancer.
(9) Combination of 18 w/w% to 22 w/w% of additive and 18 w/w% to 22 w/w% of permeation enhancer.
(10) Combination of 20 w/w% of additive and 20 w/w% of permeation enhancer.

In one embodiment of the present invention, a more preferable combination of an additive and a permeation enhancer is a combination of medium-chain triglyceride and a permeation enhancer.

In another embodiment of the present invention, a preferable combination of an additive and a permeation enhancer is a combination of a non-volatile component and C1-C4 alkyl lactate. A more preferable combination is a combination of medium-chain triglyceride and ethyl lactate.

As a combination of medium-chain triglyceride and ethyl lactate contained in the topical preparation of the present invention, for example, the following combinations are preferable.
(1) Combination of 1 w/w% to 30 w/w% of medium-chain triglyceride and 1 w/w% to 30 w/w% of ethyl lactate.
(2) Combination of 5 w/w% to 22 w/w% of medium-chain triglyceride and 5 w/w% to 22 w/w% of ethyl lactate.
(3) Combination of 10 w/w% to 22 w/w% of medium-chain triglyceride and 5 w/w% to 22 w/w% of ethyl lactate.
(4) Combination of 18 w/w% to 22 w/w% of medium-chain triglyceride and 5 w/w% to 22 w/w% of ethyl lactate.
(5) Combination of 20 w/w% of medium-chain triglyceride and 5 w/w% to 22 w/w% of ethyl lactate.
(6) Combination of 5 w/w% to 22 w/w% of medium-chain triglyceride and 10 w/w% to 22 w/w% of ethyl lactate.
(7) Combination of 5 w/w% to 22 w/w% of medium-chain triglyceride and 18 w/w% to 22 w/w% of ethyl lactate.
(8) Combination of 5 w/w% to 22 w/w% of medium-chain triglyceride and 20 w/w% of ethyl lactate.
(9) Combination of 18 w/w% to 22 w/w% of medium-chain triglyceride and 18 w/w% to 22 w/w% of ethyl lactate.
(10) Combination of 20 w/w% of medium-chain triglyceride and 20 w/w% of ethyl lactate.

In another embodiment of the present invention, the preparation of the present invention is a non-coating topical therapeutic agent for nail diseases. The non-coating topical therapeutic agent for nail diseases of one embodiment of the present invention contains a non-volatile component and a permeation enhancer and is substantially free of a film-forming substance.

The "disodium edetate hydrate" is a metal chelating agent also called an ethylenediaminetetraacetic acid disodium salt and abbreviated as EDTA. Generally, disodium edetate hydrate is added as a stabilizer of preparations. It is not questioned whether disodium edetate hydrate is added as long as the stability can be secured in the pharmaceutical formulation in the present invention. When it is added, the content of disodium edetate hydrate in the preparation of the present invention is preferably 0.0001 w/w% to 0.001 w/w% from the aspect of the solubility of disodium edetate hydrate. For example, the preparation of the present invention may contain about 0.00025 w/w% of disodium edetate hydrate.

The preparation of the present invention may contain water as long as the stability of the preparation can be secured. For example, the preparation of the present invention may contain about 1 w/w% of water.

The "preparation property" in the present specification refers to physicochemical properties suitable for a topical therapeutic agent for tinea unguium. The "preparation property" includes properties that should be generally provided as a pharmaceutical preparation to be applied topically, such as solubility and stability. For example, in the case of an antifungal active substance having low solubility, the antifungal active substance may be precipitated on the applied site after application. As the "preparation properties" required for a topical pharmaceutical preparation, preparation properties that prevent precipitation of the antifungal active substance even after application and make the drug stay in the affected part are preferable. In addition, it is preferable to have stable preparation properties that can be stored for a long term.

The "preparation property" also includes unique physicochemical properties particularly suitable for topical therapeutic agents for tinea unguium. For example, a preparation of a topical therapeutic agent for tinea unguium preferably has preparation properties with a low surface tension in order to penetrate an antifungal active substance in between a nail and a nail bed. For example, a preparation with a surface tension at 23°C±1°C of not more than 40 mN/m is preferable, and a preparation with a surface tension of not more than 30 mN/m is more preferable. The surface tension can be measured, for example, using an automatic surface tensiometer (e.g., model: K12, manufactured by KRUSS).

The preparation of the present invention is a preparation satisfying these preparation properties.

The percentage (%) in the present specification refers to a mass percentage (w/w%) unless particularly described.

A preferable embodiment of the present invention is explained below. A preferable embodiment of the present invention is, for example, the following topical preparation.
(1A) A topical preparation that is a therapeutic agent for tinea unguium, containing:
   a) 1 w/w% to 30 w/w% of an antifungal active substance,
   b) 35 w/w% to 65 w/w% of ethanol,
   c) a medium-chain triglyceride, and
   d) 18 w/w% to 22 w/w% of ethyl lactate.
(2A) The topical preparation of (1A), wherein a content of the medium-chain triglyceride is 1 w/w% to 30 w/w%.
(3A) The topical preparation of (1A) or (2A), wherein the content of the medium-chain triglyceride is 5 w/w% to 22 w/w%.
(4A) The topical preparation of any of (1A) to (3A), wherein the content of the medium-chain triglyceride is 18 w/w% to 22 w/w%.
(5A) The topical preparation of any of (1A) to (4A), wherein the medium-chain triglyceride is tri(caprylic acid/capric acid) glyceride.
(6A) The topical preparation of any of (1A) to (5A), wherein an acid value of ethyl lactate is 0 to 0.5.
(7A) The topical preparation of any of (1A) to (6A), wherein the antifungal active substance is a compound represented by the formula (I): 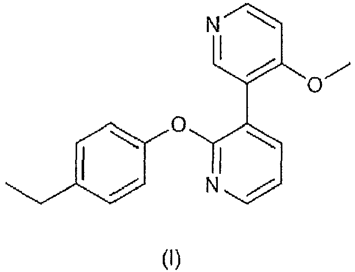 or a salt thereof.
(8A) The topical preparation of any of (1A) to (6A), wherein the antifungal active substance is efinaconazole or a salt thereof.
(9A) The topical preparation of any of (1A) to (6A), wherein the antifungal active substance is luliconazole or a salt thereof.

In another embodiment of the present invention, a preferable embodiment is, for example, the following topical preparation.
(1B) A topical preparation that is a therapeutic agent for tinea unguium, containing:
   a) 1 w/w% to 30 w/w% of an antifungal active substance,
   b) 35 w/w% to 65 w/w% of ethanol,
   c) 18 w/w% to 22 w/w% of a medium-chain triglyceride, and
   d) ethyl lactate.
(2B) The topical preparation of (1B), wherein a content of the ethyl lactate is 1 w/w% to 30 w/w%.
(3B) The topical preparation of (1B) or (2B), wherein the content of the ethyl lactate is 5 w/w% to 22 w/w%.
(4B) The topical preparation of any of (1B) to (3B), wherein the content of the ethyl lactate is 18 w/w% to 22 w/w%.
(5B) The topical preparation of any of (1B) to (4B), wherein the medium-chain triglyceride is tri(caprylic acid/capric acid) glyceride.
(6B) The topical preparation of any of (1B) to (5B), wherein an acid value of ethyl lactate is 0 to 0.5.
(7B) The topical preparation of any of (1B) to (6B), wherein the antifungal active substance is a compound represented by the formula (I): 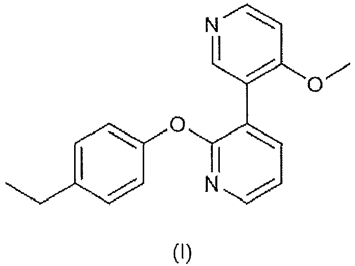 or a salt thereof.
(8B) The topical preparation of any of (1B) to (6B), wherein the antifungal active substance is efinaconazole or a salt thereof.
(9B) The topical preparation of any of (1B) to (6B), wherein the antifungal active substance is luliconazole or a salt thereof.

In another embodiment of the present invention, a preferable embodiment is, for example, the following topical preparation.
(1C) A topical preparation that is a therapeutic agent for tinea unguium, containing:
   a) 1 w/w% to 30 w/w% of an antifungal active substance, and
   b) a permeation enhancer,
   wherein the above-mentioned antifungal active substance is a compound represented by the formula (I): 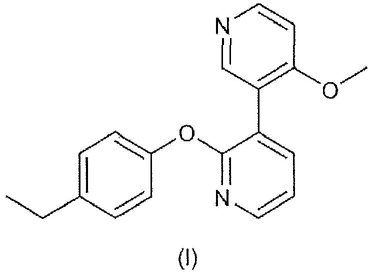 or a salt thereof.
(2C) The topical preparation of (1C) further containing c) an additive.
(3C) The topical preparation of (2C), wherein the additive is a non-volatile component.
(4C) The topical preparation of (2C) or (3C), wherein the additive is a medium-chain triglyceride.
(5C) The topical preparation of any of (2C) to (4C), wherein a content of the additive is 1 w/w% to 30 w/w%.
(6C) The topical preparation of any of (2C) to (5C), wherein a content of the additive is 5 w/w% to 22 w/w%.
(7C) The topical preparation of any of (2C) to (6C), wherein a content of the additive is 10 w/w% to 22 w/w%.
(8C) The topical preparation of any of (1C) to (7C), further containing d) a volatile component.
(9C) The topical preparation of (8C), wherein the volatile component is ethanol.
(10C) The topical preparation of (8C) or (9C), wherein a content of the volatile component is 35 w/w% to 85 w/w%.
(11C) The topical preparation of any of (8C) to (10C), wherein the content of the volatile component is 35 w/w% to 65 w/w%.
(12C) The topical preparation of any of (1C) to (11C), wherein the permeation enhancer is a lower (C1-C4) alkyl ester of hydroxyl acid.
(13C) The topical preparation of any of (1C) to (12C), wherein the permeation enhancer is a lower (C1-C4) alkyl ester of aliphatic hydroxyl acid.
(14C) The topical preparation of any of (1C) to (13C), wherein the permeation enhancer is C1-C4 alkyl lactate.
(15C) The topical preparation of any of (1C) to (14C), wherein the permeation enhancer is ethyl lactate.
(16C) The topical preparation of any of (1C) to (15C), wherein the permeation enhancer is ethyl lactate having an acid value of not more than 0.5.
(17C) The topical preparation of any of (1C) to (16C), wherein a content of the permeation enhancer is 1 w/w% to 30 w/w%.
(18C) The topical preparation of any of (1C) to (17C), wherein the content of the permeation enhancer is 5 w/w% to 22 w/w%.
(19C) The topical preparation of any of (1C) to (18C), wherein the content of the permeation enhancer is 10 w/w% to 22 w/w%.
(20C) The topical preparation of any of (1C) to (19C) which is a non-coating topical preparation.
(21C) The topical preparation of any of (1C) to (20C) having a surface tension at 23°C±1°C of not more than 40 mN/m.

In another embodiment of the present invention, a preferable embodiment is, for example, the following topical preparation.
(1D) A topical preparation that is a therapeutic agent for a nail disease, containing:
   a) a physiologically active substance,
   b) a non-volatile component, and
   c) a permeation enhancer.
(2D) The topical preparation of (1D) further containing d) a volatile component.
(3D) The topical preparation of (1D) or (2D), wherein the permeation enhancer is C1-C4 alkyl lactate.
(4D) The topical preparation of any of (1D) to (3D), wherein the permeation enhancer is ethyl lactate.
(5D) The topical preparation of any of (1D) to (4D), wherein the non-volatile component is a medium-chain triglyceride.
(6D) The topical preparation of any of (1D) to (5D) which is a non-coating topical preparation.
(7D) The topical preparation of any of (1D) to (6D) having a surface tension at 23°C±1°C of not more than 40 mN/m.
(8D) The topical preparation of any of (1D) to (7D), wherein the nail disease is tinea unguium and the physiologically active substance is an antifungal active substance.

### [Example]

The present invention is explained more specifically in the following by referring to Examples.

In the following Examples and Comparative Examples, the medium-chain triglyceride used was MIGLYOL 810 (manufactured by IOI Oleo GmbH). In the following Example and Comparative Example (excluding Comparative Example 5), the acid value of ethyl lactate was not more than 0.5, and ethyl lactate manufactured by Musashino Chemical Laboratory, Ltd. was used.

### <Test Example 1>

### <Test relating to preparation property (10% preparation)>

The preparations having the formulations shown in Table 1 (Examples 1a - 5a) were respectively prepared. That is, components excluding compound A, disodium edetate hydrate and purified water were mixed by stirring. Separately, disodium edetate hydrate (0.075 g) was dissolved in purified water (300 g) to prepare purified water containing 0.025 w/w% of disodium edetate hydrate. The purified water was added to the above mixture and mixed by stirring. Respective additives were blended and dissolved, to which compound A was added and dissolved by stirring to give a preparation. The presence or absence of crystal precipitation was confirmed for the preparations of the present invention. Examples 1a - 5a were applied on a glass plate and the presence or absence of crystal precipitation at room temperature up to day 3 was evaluated (Table 1). Concurrently, surface tension was also evaluated.

**[Table 1]**

| <Test relating to preparation property (10% preparation)> | | | | | |
|---|---|---|---|---|---|
| component | Ex. 1a | Ex. 2a | Ex. 3a | Ex. 4a | Ex. 5a |
| compound A | 10 w/w% | 10 w/w% | 10 w/w% | 10 w/w% | 10 w/w% |
| medium-chain triglyceride | 20 w/w% | 20 w/w% | 20 w/w% | 5 w/w% | 10 w/w% |
| ethyl lactate | 5 w/w% | 10 w/w% | 20 w/w% | 20 w/w% | 20 w/w% |
| purified water (containing disodium edetate hydrate*) | 1 w/w% | | | | |
| anhydrous ethanol | 64 w/w% | 59 w/w% | 49 w/w% | 64 w/w% | 59 w/w% |
| presence or absence of crystal precipitation | ○ | ○ | ○ | ○ | ○ |
| surface tension (23±1°C) | 24.4 mN/m | 24.8 mN/m | 25.5 mN/m | 24.2 mN/m | 24.8 mN/m |

| | | | | | |
|---|---|---|---|---|---|
| ○: Crystal precipitation of compound A is not observed. * : The net content of disodium edetate hydrate contained in the preparations of Examples 1a to 5a is 0.00025 w/w%. | | | | | |

In the case of a topical preparation containing a volatile component (e.g., anhydrous ethanol) and an antifungal active substance (e.g., compound A), when a given amount of a non-volatile component is not present, crystal precipitation of an antifungal active substance is feared and an adverse influence is exerted on the nail permeability and efficacy expression. As shown in Table 1, the preparations described in Table 1 containing medium-chain triglyceride which is a non-volatile component did not show crystal precipitation of the antifungal active substance even after application. The surface tension of these preparations was not more than 30 mN/m, and it was confirmed that they were low surface tension preparations and it was suggested that they have preparation properties suitable for topical therapeutic agents for tinea unguium. The surface tension was measured using an automatic surface tensiometer (model: K12, manufactured by KRUSS).

From the above study, it was shown that the formulations described in Table 1 have preparation properties suitable for non-coating topical therapeutic agents for tinea unguium.

### <Test Example 2>

### <Nail permeation test>

Comparative Examples 1, 2, and 3 shown in Table 3 were prepared similarly to Test Example 1.

The evaluation method of the nail permeation was applied one time to bovine ungula (thickness 250 - 350 µm) at 5 µL/0.071 cm². Franz diffusion cell was placed in an incubator at 32°C, a receptor solution stirred by a stirrer was partly collected over time up to day 14. The drug concentration of the collected receptor solution was measured by LC-MS/MS and the nail permeation amount was calculated.

By this test, it was found that a formulation adding ethyl lactate alone (Comparative Example 2 and Comparative Example 3) did not show a permeation enhancing effect, and the nail permeation amount was smaller than Comparative Example 1. On the other hand, surprisingly, when 5 w/w% - 20 w/w% of ethyl lactate was further added to Comparative Example 1, the nail permeation amount was strikingly improved (Examples 1a - 3a). The permeation enhancing effect by the addition of ethyl lactate was also found even when medium-chain triglyceride was 5 w/w% or 10 w/w% (Examples 4a, 5a). That is, in all formulations (Examples 1a - 5a) containing both medium-chain triglyceride and ethyl lactate, the nail permeation amount was strikingly improved.

From the above results, it was found that the formulation of the present invention is extremely preferable as a formulation of a topical therapeutic agent for tinea unguium since it contains both medium-chain triglyceride and ethyl lactate, which improves a nail permeation amount of an antifungal active substance.

**[Table 2]**

| <Nail permeation test> | | | | | | | | |
|---|---|---|---|---|---|---|---|---|
| component | Ex. 1a | Ex. 2a | Ex. 3a | Ex. 4a | Ex. 5a | Comp. Ex. 1*² (standard formulation) | Comp. Ex. 2*² | Comp. Ex. 3*² |
| nail permeation amount µg/cm²) | 17.0 | 17.7 | 17.7 | 22.7 | 26.2 | 11.0 | 9.77 | 6.08 |
| Δ nail permeation amount *¹ | +6.0 | +6.7 | +6.7 | +11.7 | +15.2 | 0 | -1.23 | -4.92 |

| | | | | | | | | |
|---|---|---|---|---|---|---|---|---|
| *1 Difference in nail permeation amount µg/cm²) from Comparative Example 1 was taken. | | | | | | | | |

**[Table 3]**

| *2 Comparative Examples 1, 2 and 3 | | | |
|---|---|---|---|
| component | Comp. Ex. 1 | Comp. Ex. 2 | Comp. Ex. 3 |
| compound A | 10 w/w% | 10 w/w% | 10 w/w% |
| medium-chain triglyceride | 20 w/w% | - | - |
| ethyl lactate | - | 5 w/w% | 20 w/w% |
| purified water (containing disodium edetate hydrate*) | 1 w/w% | | |
| anhydrous ethanol | 69 w/w% | 84 w/w% | 69 w/w% |

| | | | |
|---|---|---|---|
| * : The net content of disodium edetate hydrate in the preparations of Comparative Examples 1 to 3 is 0.00025 w/w%. | | | |

### <Test Example 3>

### <Evaluation of treatment effect in tinea unguium model>

Examples 3b and 5b shown in Table 5 were prepared similarly to Test Example 1.

An evaluation method of the effect in tinea unguium model induced fungal nail disease in 5-week-old Hartley guinea pigs. A suspension of Trichophyton mentagrophytes SM-110 (1x10⁸ cells/mL) was inoculated into the interdigital skin of sole and hindpaw, and then the entire paw was covered with a bandage. The bandage was removed 14 days after the fungal inoculation and bred until day 28 of infection. The formulation shown in Table 4 was applied for 28 days to the infected nail. The infected nail was removed from the feet 7 days after the final treatment and minced with scissors. The nail was placed on a glass homogenizer, PBS (phosphoric acid buffered brine) containing 0.25 w/v% swine pancreas trypsin was added at 1 mL per 50 mg of wet nail weight, and the nail was homogenized. The homogenate solution was left standing at 37°C for 1 hr. 100 µL of the nail homogenate solution or a diluted solution thereof was spread on a GPLP agarose medium containing an antibiotic, and cultured at 30°C for 14 days. After culturing, the fungal colonies that appeared on the medium were counted and the number of fungal colony formation units (CFU) in the nail was calculated.

From the results of this test, the preparation of the present invention containing both medium-chain triglyceride and ethyl lactate showed efficacy (the treatment effect on tinea unguium) equivalent to that of a CLENAFIN (registered trade mark) topical solution 10% for nail (trade name) (containing efinaconazole 10 w/w%) which is commercially available as an topical therapeutic agent for tinea unguium. Examples of a particularly preferable formulation include a formulation containing both 20 w/w% medium-chain triglyceride and 20 w/w% ethyl lactate (Examples 3a, 3b).

**[Table 4]**

| <Evaluation of treatment effect in tinea unguium model> | | | | | | | |
|---|---|---|---|---|---|---|---|
| component | Ex. 1a | Ex. 2a | Ex. 3a | Ex. 3b*¹ | Ex. 5b*¹ | CLENAFIN | positive control |
| nail fungi count (Log CFU/foot) | 3.42 ±0.54 | 3.16 ±0.28 | 2.87 ±0.51 | 2.75 ±0.59 | 3.15 ±0.65 | 2.59 ±0.70 | 4.59 ±0.25 |

**[Table 5]**

| *1 Examples 3b and 5b | | |
|---|---|---|
| component | Ex. 3b | Ex. 5b |
| compound A | 10 w/w% | 10 w/w% |
| medium-chain triglyceride | 20 w/w% | 10 w/w% |
| ethyl lactate | 20 w/w% | 20 w/w% |
| anhydrous ethanol | 50 w/w% | 60 w/w% |

### <Test Example 4>

### <Test relating to preparation property (presence or absence of crystal precipitation) (20% preparation)>

The preparations of the present invention having a compound A content of 20 w/w% (Examples 3c - 5c) were prepared by a method similar to that in Test Example 1, and the presence or absence of crystal precipitation was evaluated by a method similar to that in Test Example 1 (Table 6). Examples 3c - 5c were preparations containing a high concentration of a antifungal active substance (20 w/w%), but did not show crystal precipitation.

That is, it was found that a formulation containing 20 w/w% ethyl lactate and 5 w/w% - 20 w/w% medium-chain triglyceride in combination showed high solubility of antifungal active substances and is preferable as a non-coating preparation.

**[Table 6]**

| <Test relating to preparation property (presence or absence of crystal precipitation) (20% preparation)> | | | |
|---|---|---|---|
| component | Ex. 3c | Ex. 4c | Ex. 5c |
| compound A | 20 w/w% | 20 w/w% | 20 w/w% |
| medium-chain triglyceride | 20 w/w% | 5 w/w% | 10 w/w% |
| ethyl lactate | 20 w/w% | 20 w/w% | 20 w/w% |
| purified water (containing disodium edetate hydrate*) | 1 w/w% | | |
| anhydrous ethanol | 39 w/w% | 54 w/w% | 49 w/w% |
| presence or absence of crystal precipitation | ○ | ○ | ○ |

| | | | |
|---|---|---|---|
| ○ : Crystal precipitation of compound A was not observed. * : The net content of disodium edetate hydrate contained in the preparations of Examples 3c to 5c was 0.00025 w/w%. | | | |

### <Test Example 5>

### <Test relating to amount of disodium edetate hydrate to be added>

Examples 3d and 3e, and Comparative Example 4 shown in Table 7 were prepared by a method similar to that in Test Example 1. By changing the concentration of sodium edetate hydrate in the purified water in the step of preparing purified water containing sodium edetate hydrate of Test Example 1, the content of sodium edetate hydrate in the preparation was adjusted to be the amount shown in Table 7.

The presence or absence of addition of disodium edetate hydrate to the preparation according to the present invention is not questioned as long as the antifungal active substance can be stably stored. Depending on the stability of the antifungal active substance in the preparation, disodium edetate hydrate can be added as necessary. However, addition of a large amount of disodium edetate hydrate causes precipitation of disodium edetate hydrate. Thus, the upper limit thereof was studied.

That is, the respective preparations (Examples 3b, 3d, 3e and Comparative Example 4) were evaluated for the presence or absence of precipitation of disodium edetate hydrate (one day at 5°C → one day at room temperature → one day at 5°C → one day at room temperature → 3 days at 5°C) (Table 7). As a result, Comparative Example 4 showed precipitation of disodium edetate hydrate. Thus, it was found that disodium edetate hydrate is preferably added at 0.001 w/w% at maximum.

**[Table 7]**

| <Test relating to amount of disodium edetate hydrate to be added> | | | | |
|---|---|---|---|---|
| component | Ex. 3b | Ex. 3d | Ex. 3e | Comp. Ex. 4 |
| compound A | 10 w/w% | 10 w/w% | 10 w/w% | 10 w/w% |
| medium-chain triglyceride | 20 w/w% | 20 w/w% | 20 w/w% | 20 w/w% |
| ethyl lactate | 20 w/w% | 20 w/w% | 20 w/w% | 20 w/w% |
| purified water (containing disodium edetate hydrate) | - | 1 w/w% | 1 w/w% | 1 w/w% |
| anhydrous ethanol | 50 w/w% | 49 w/w% | 49 w/w% | 49 w/w% |
| presence or absence of precipitation | ○ | ○ | ○ | × |
| net content of disodium edetate hydrate contained in preparation | - | 0.0001 w/w% | 0.001 w/w% | 0.005 w/w% |

| | | | | |
|---|---|---|---|---|
| ○ : Precipitation of disodium edetate hydrate was not found. × : Precipitation of disodium edetate hydrate was found. | | | | |

### <Test Example 6>

### <Influence of acid value of ethyl lactate on stability>

The formulations shown in Table 8 were prepared similarly to Test Example 1.

The preparations were placed and sealed in a glass ampoule, stored at 60°C (shading) for 4 weeks, and subjected to a purity test (related substances derived from compound A) measurement by high performance liquid chromatography. The results thereof are shown in Table 9.

It was found that an increase in the related substances can be suppressed when the acid value is not more than 0.5. The results clarified that a preferable acid value of ethyl lactate was not more than 0.5.

**[Table 8]**

| | | |
|---|---|---|
| <Stability of each preparation having different acid value of ethyl lactate> | | |

| component | Ex. 3b | Comp. Ex. 5 |
|---|---|---|
| compound A | 10 w/w% | 10 w/w% |
| medium-chain triglyceride | 20 w/w% | 20 w/w% |
| ethyl lactate | 20 w/w% (acid value not more than 0.5) | 20 w/w% (acid value 1.0) |
| anhydrous ethanol | 50 w/w% | 50 w/w% |

| | | |
|---|---|---|
| acid value: Value by Simple Pack for fats and oils deterioration measurement Acid Value 1 manufactured by SIBATA SCIENTIFIC TECHNOLOGY LTD. | | |

**[Table 9]**

| | | |
|---|---|---|
| Results of purity test (related substances) | | |
| amount of increase in related substances after 60°C (shading), 4 week storage | Ex. 3b (acid value of ethyl lactate, not more than 0.5) | Comp. Ex. 5 (acid value of ethyl lactate, 1.0) |
| | 0.08% | 0.98% |

### <Test Example 7>

### <Nail permeation test using other antifungal active substances>

By a method similar to that in Test Example 1, the formulations shown in Table 10 (Examples 6 - 10), the formulations shown in Table 11 (Examples 11 - 15) and the formulations shown in Table 12 (Comparative Examples 1E, 3E, 1L, 3L) were prepared.

**[Table 10]**

| <Formulation Example using efinaconazole> | | | | | |
|---|---|---|---|---|---|
| component | Ex. 6 | Ex. 7 | Ex. 8 | Ex. 9 | Ex. 10 |
| efinaconazole | 10 w/w% | 10 w/w% | 10 w/w% | 10 w/w% | 10 w/w% |
| medium-chain triglyceride | 20 w/w% | 20 w/w% | 20 w/w% | 5 w/w% | 10 w/w% |
| ethyl lactate | 5 w/w% | 10 w/w% | 20 w/w% | 20 w/w% | 20 w/w% |
| purified water (containing disodium edetate hydrate*) | 1 w/w% | | | | |
| anhydrous ethanol | 64 w/w% | 59 w/w% | 49 w/w% | 64 w/w% | 59 w/w% |

| | | | | | |
|---|---|---|---|---|---|
| * : The net content of disodium edetate hydrate contained in the preparations of Examples 6 to 10 was 0.00025 w/w%. | | | | | |

**[Table 11]**

| <Formulation Example using luliconazole> | | | | | |
|---|---|---|---|---|---|
| component | Ex. 11 | Ex. 12 | Ex. 13 | Ex. 14 | Ex. 15 |
| luliconazole | 3 w/w% | 3 w/w% | 3 w/w% | 3 w/w% | 3 w/w% |
| medium-chain triglyceride | 20 w/w% | 20 w/w% | 20 w/w% | 5 w/w% | 10 w/w% |
| ethyl lactate | 5 w/w% | 10 w/w% | 20 w/w% | 20 w/w% | 20 w/w% |
| purified water (containing disodium edetate hydrate*) | 1 w/w% | | | | |
| anhydrous ethanol | 71 w/w% | 66 w/w% | 56 w/w% | 71 w/w% | 66 w/w% |

| | | | | | |
|---|---|---|---|---|---|
| * : The net content of disodium edetate hydrate contained in the preparations of Examples 11 to 15 was 0.00025 w/w%. | | | | | |

**[Table 12]**

| component | Comp. Ex. 1E | Comp. Ex. 3E | Comp. Ex. 1L | Comp. Ex. 3L |
|---|---|---|---|---|
| efinaconazole | 10 w/w% | 10 w/w% | - | - |
| luliconazole | - | - | 3 w/w% | 3 w/w% |
| medium-chain triglyceride | 20 w/w% | - | 20 w/w% | - |
| ethyl lactate | - | 20 w/w% | - | 20 w/w% |
| purified water (containing disodium edetate hydrate*) | 1 w/w% | | | |
| anhydrous ethanol | 69 w/w% | 69 w/w% | 76 w/w% | 76 w/w% |

| | | | | |
|---|---|---|---|---|
| * : The net content of disodium edetate hydrate contained in the preparations of. Comparative Examples 1E, 3E, 1L and 3L was 0.00025 w/w%. | | | | |

The formulations described in Table 10, Table 11 and Table 12 were evaluated for nail permeability by a method similar to that in Test Example 2. The results thereof are shown in Table 13 and Table 14. It was found that the nail permeation amount was improved in both cases of efinaconazole and luliconazole by containing both medium-chain triglyceride and ethyl lactate.

**[Table 13]**

| <Nail permeation test: efinaconazole> | | | | | | | |
|---|---|---|---|---|---|---|---|
| component | Ex. 6 | Ex. 7 | Ex. 8 | Ex. 9 | Ex. 10 | Comp. Ex. 1E (standard formulation) | Comp. Ex. 3E |
| nail permeation amount µg/cm²) | 10.4 | 5.13 | 6.23 | 8.82 | 10.5 | 4.19 | 3.67 |
| Δ nail permeation amount*¹ | +6.21 | +0.94 | +2.04 | +4.63 | +6.31 | 0 | -0.52 |

| | | | | | | | |
|---|---|---|---|---|---|---|---|
| *1 Difference in nail permeation amount µg/cm²) from Comparative Example 1E was taken. | | | | | | | |

**[Table 14]**

| <Nail permeation test: luliconazole> | | | | | | | |
|---|---|---|---|---|---|---|---|
| component | Ex. 11 | Ex. 12 | Ex. 13 | Ex. 14 | Ex. 15 | Comp. Ex. 1L (standard formulation) | Comp. Ex. 3L |
| nail permeation amount µg/cm²) | 1.25 | 1.22 | 1.40 | 1.14 | 1.34 | 0.894 | 0.622 |
| Δ nail permeation amount*¹ | +0.356 | +0.326 | +0.506 | +0.246 | +0.446 | 0 | -0.272 |

| | | | | | | | |
|---|---|---|---|---|---|---|---|
| *1 Difference in nail permeation amount µg/cm²) from Comparative Example 1L was taken. | | | | | | | |

By a method similar to that in Test Example 1, the formulations shown in Table 10 (Examples 6 - 10) were prepared and subjected to evaluation of the presence or absence of crystal precipitation by a method similar to that in Test Example 1.

None of the preparations showed crystal precipitation of efinaconazole, and it was shown that the preparation of the present invention containing medium-chain triglyceride and ethyl lactate is a preparation preferable as a non-coating topical therapeutic agent for tinea unguium even when the antifungal active substance is efinaconazole.

### [Industrial Applicability]

According to the present invention, a topical therapeutic agent for tinea unguium can be provided, which is superior in the aspects of nail permeability, efficacy, and preparation properties, and can be applied clinically.

This application is based on patent application No. 2017-209136 filed in Japan, and the contents thereof are encompassed in full herein.

## Claims

1. A topical preparation that is a therapeutic agent for tinea unguium, comprising:
a) an antifungal active substance,
b) a volatile component,
c) a medium-chain triglyceride, and
d) ethyl lactate.

2. The topical formulation according to claim 1, wherein an amount of the ethyl lactate is 1 w/w% to 30 w/w%.

3. The topical preparation according to claim 1 or 2, wherein an amount of the ethyl lactate is 5 w/w% to 22 w/w%.

4. The topical preparation according to any one of claims 1 to 3, wherein an amount of the ethyl lactate is 10 w/w% to 22 w/w%.

5. The topical preparation according to any one of claims 1 to 4, wherein an amount of the medium-chain triglyceride is 1 w/w% to 30 w/w%.

6. The topical preparation according to any one of claims 1 to 5, wherein an amount of the medium-chain triglyceride is 5 w/w% to 22 w/w%.

7. The topical preparation according to any one of claims 1 to 6, wherein an amount of the medium-chain triglyceride is 10 w/w% to 22 w/w%.

8. The topical preparation according to any one of claims 1 to 7, wherein an amount of the antifungal active substance is 1 w/w% to 30 w/w%.

9. The topical preparation according to any one of claims 1 to 8, wherein an amount of the antifungal active substance is 1 w/w% to 15 w/w%.

10. The topical preparation according to any one of claims 1 to 9, wherein the volatile component is ethanol.

11. The topical preparation according to claim 10, wherein an amount of the ethanol is 35 w/w% to 85 w/w%.

12. The topical preparation according to any one of claims 1 to 11, wherein an acid value of the ethyl lactate is 0 to 0.5.

13. The topical preparation according to any one of claims 1 to 12, further comprising e) disodium edetate hydrate.

14. The topical preparation according to claim 13, wherein an amount of the disodium edetate hydrate is 0.0001 w/w% to 0.001 w/w%.

15. The topical preparation according to any one of claims 1 to 14, wherein the antifungal active substance is a compound represented by the formula (I): 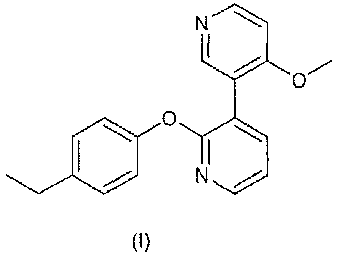 or a salt thereof.

16. The topical preparation according to any of claims 1 to 14, wherein the antifungal active substance is a compound represented by the formula (II): 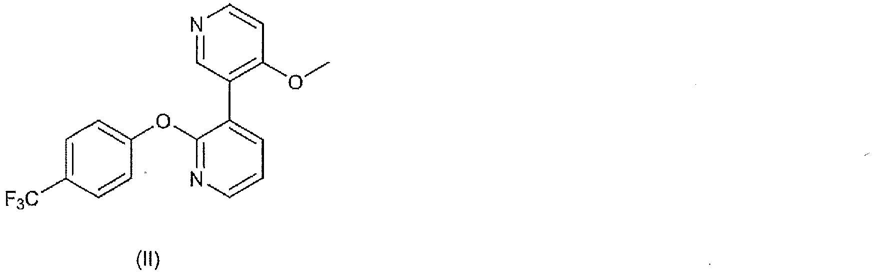 or a salt thereof.

17. The topical preparation according to any of claims 1 to 14, wherein the antifungal active substance is efinaconazole or a salt thereof.

18. The topical preparation according to any of claims 1 to 14, wherein the antifungal active substance is luliconazole or a salt thereof.
